(19)

Europäisches
Patentamt
European
Patent Office
Office européen
des brevets

(11) **EP 4 173 514 A1**

(12) **EUROPEAN PATENT APPLICATION**

(43) Date of publication:
**03.05.2023 Bulletin 2023/18**

(21) Application number: **21306522.0**

(22) Date of filing: **29.10.2021**

(51) International Patent Classification (IPC):
*A43B 3/34* (2022.01)     *A43B 3/44* (2022.01)
*A61B 5/11* (2006.01)     *A61B 5/00* (2006.01)
*G06V 40/20* (2022.01)     *A43B 17/00* (2006.01)

(52) Cooperative Patent Classification (CPC):
**A43B 3/34; A43B 3/44; A43B 17/00; A61B 5/112;
A61B 5/6807; A61B 5/6829; G06V 40/25**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Feetme
75019 Paris (FR)**

(72) Inventors:
• **HUANG, Piao
75019 Paris (FR)**
• **MOSTOVOV, Andrey
75019 Paris (FR)**

(74) Representative: **Icosa
83 avenue Denfert-Rochereau
75014 Paris (FR)**

(54)     **TOE OFF DETECTION METHOD WITH A PRESSURE SENSOR**

(57)     A method (100) for determining toe off using a plurality of sensing cells, the method comprising: during the discharge period, collecting (130) the measured signal from each sensing cell, outputting (156, 160), for each sensing cell, a null output if said signal is lower than said predetermined cell calibration threshold or the measured signal if said signal is larger than or equal to a predetermined cell calibration threshold, triggering (190) output of the toe off when the total signal is lower than a predetermined toe off threshold.

**FIG. 4**

EP 4 173 514 A1

**Description**

**FIELD OF INVENTION**

The present invention relates to a method for determining toe off. More precisely, the present invention relates to a method for determining toe off with an insole comprising a plurality of sensing cells.

The present invention also relates to a computer program product to carry out the steps of the method.

**BACKGROUND OF INVENTION**

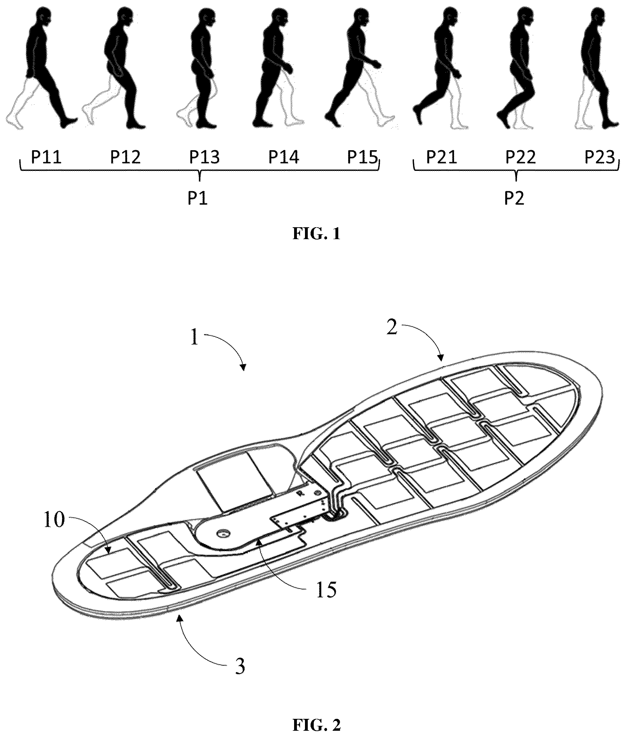

In various fields, such as medical fields or sport training, it is often desirable to determine, during the gait cycle, the time at which a foot of a user does not contact the ground anymore allowing the determination of the end of the stance phase and the beginning of the swing phase (respectively P1 and P2 in figure 1). This time is known as toe off.

To do so, sensing cells are often used. In order to obtain the toe off as precisely as possible, the signal measured by each sensing cell must reflect exactly the pressure applied to said sensing cell. However, dielectric materials usually used in sensing cells are subject to latency, also called elastic recovery. This latency is a phenomenon occurring during the decay of the pressure applied to a sensing cell and manifesting itself as a signal decay from the cell which is delayed compared to the variation of the pressure. Therefore, the sensing cell still measures a signal when the pressure applied to this cell is null. This phenomenon thus leads to a time delay in the order of several milliseconds when measuring the variation of pressure from sensing cells. In the case of the gait, latency occurs during the pre-swing phase (P15 in figure 1), i.e., just before the foot of the user is off the ground. The time delay due to the latency thus leads to a loss of precision for the determination of the time at which the foot of the user does not contact the ground anymore (the toe off).

Known methods for the determination of the toe off using sensing cells are not satisfactory.

Indeed, several known methods use thousands of sensing cells to capture the geometry and relative arrangement of each footfall as a function of time. Moreover, several known methods require combining data captured from various sensors such as pressure sensors, camera, gyroscope, angular velocity sensors... making the method complex, time consuming and tedious. Moreover, these systems are expensive and can only be used within clinical and controlled laboratory environments. In addition, since the analysis is performed in a confined and controlled environment, patients may not exhibit their natural movement patterns because a limited number of strides/gestures can be observed.

A purpose of the invention is to provide a toe off detection method that is more precise, more reliable or that may be performed at lower cost outside controlled laboratory environments.

**SUMMARY**

To this end, the present invention relates to a method for determining toe off with an insole comprising a controller and a plurality of sensing cells, the method comprising:

- Identifying a discharge period,
- During the discharge period:

    o Collecting by the controller a measured signal from each sensing cell,
    ◦ Outputting by the controller, for each sensing cell:

        ■ The signal if said signal is larger than or equal to a predetermined cell calibration threshold,
        ■ A null output if said signal is lower than said predetermined cell calibration threshold,

    o Computing, from the overall output signals, a total signal of the sensing cells, and
    ◦ Triggering output of the toe off when the total signal is lower than a predetermined toe off threshold,

wherein the predetermined cell calibration thresholds are values determined during the calibration phase for each sensing cell as the signal measured by said sensing cell when an applied load becomes null during an unloading phase.

Indeed, the method being performed only during the discharge period, this allows to reduce the energy consumption of the insole compared to known methods performed during the whole gait cycle.

Moreover, outputting, for each sensing cell, a null output if a signal is lower than a predetermined cell calibration threshold allows to suppress most of the noisy signal produced, among other things, by the latency of the sensing cells allowing a method which is more precise.

Furthermore, triggering output of the toe off when the total signal is lower than a predetermined toe off threshold allows to correct from any possible remaining noisy signal.

Finally, the method is based only on sensing cells comprised in an insole. This leads to a method which is cheaper than known methods allowing performing the method outside controlled laboratory environments.

According to another advantageous aspect of the invention, the predetermined toe off threshold corresponds to a force ranging between 5 and 40N.

According to another advantageous aspect of the invention, identifying a discharge period comprises:

- Collecting by the controller a measured signal from each sensing cell,
- Computing a total signal from the measured signals,
- Comparing the total signal and a total signal computed at a preceding instant, and
- Triggering the determination, from this comparison, of the beginning of the discharge period when the total signal computed at the preceding instant is larger than the total signal.

According to another advantageous aspect of the invention, triggering the determination of the beginning of the discharge period is performed when the total signal corresponds to a force lower than 300N.

This is advantageous because this avoids any false triggering due to the noise of each sensing cell which may imply, out of the actual discharge period, a total signal computed at the preceding instant which is larger than the total current signal.

According to another advantageous aspect of the invention, the method further comprises, before identifying a discharge period, determining heel strike comprising:

- Collecting by the controller a measured signal from each sensing cell,
- Computing a total signal from the collected signals, and
- Triggering the determination of a heel strike when the total signal exceeds a predetermined heel strike threshold.

This determination allows to increase the precision of the method. Indeed, by including this determination, the discharge period is identified only during the stance phase (i.e., when the foot is on the ground). This allows to avoid an identification of the discharge period during the swing phase due to a noise measurement from the sensing cells.

Moreover, since this allows to perform the computations for the identification of the discharge period only during the stance, this leads to a method which is less energy consuming compared to known methods performed during the whole gait cycle.

Finally, the determination of the heel strike also allows to determine the duration of the contact between the foot and the ground (i.e., the duration between the heel strike and the toe off) with a higher precision.

According to another advantageous aspect of the invention, the predetermined heel strike threshold corresponds to a force ranging between 5 and 40N.

According to another advantageous aspect of the invention, the total signal is computed as the sum of the signals outputted by the controller.

In an advantageous aspect of the invention, sensing cells measure a pressure and the total signal is the integration of a field of pressure, said field of pressure being a spatial interpolation of pressure signals outputted by the controller.

According to another advantageous aspect of the invention, at least one sensing cell is a capacitive pressure cell or a force sensing cell.

According to another advantageous aspect of the invention, the calibration phase is performed by measuring a dynamic response of sensing cell with a load decreasing from its maximum value to zero in 0.12 to 0.7 s, corresponding to usual gait behaviour.

This allows to increase the precision of the method. Indeed, measuring the dynamic response of the sensing cells allows to take the rate of load (pressure or force) variations into account since it modifies the response of the sensing cell.

According to another advantageous aspect of the invention, the sensing cells are distributed along the surface of the insole.

This allows to compute a distribution of the load along the surface of the insole with a higher precision. Moreover, the higher the number of sensing cells, the lower is the value error on the computed total signal.

According to another advantageous aspect of the invention, at least one of the sensing cells is located in the toe part of the insole.

This characteristic is advantageous in normal gait, where the toes are the last contact with the ground during the pre-swing phase. Indeed, since the toe off is the moment when the foot does not contact the ground anymore, in normal gait, locating at least one sensing cell in the toe part of the insole allows to compare the predetermined toe off threshold to a total signal to which at least the toe part of the insole contributes.

According to another advantageous aspect of the invention, at least one of the sensing cells is located in the heel part of the insole.

This characteristic is advantageous in abnormal gait, where the toes are not the last contact with the ground during the pre-swing phase. Indeed, in abnormal gait, locating at least one sensing cell in the heel part of the insole allows to compare the predetermined toe off threshold to a total signal to which at least the part of the insole other than the toe part contributes.

This invention also relates to a computer program product comprising instructions which, when the program is executed by a controller, cause the controller to carry out the steps of the method.

DEFINITIONS

In the present invention, the following terms have the following meanings:

"Discharge period" refers to a time period when the load (pressure or force) applied on sensing cells is decreasing.

"Gait cycle" or "stride" refers to the period of time between two same successive events for the same foot during gait. For instance, as illustrated in Figure 1, the period of time between two successive heel strikes of the right foot P11.

"Timestamped data" refers to data characterized by temporal information identifying the time when the data is measured.

BRIEF DESCRIPTION **OF** THE DRAWINGS

Figure 1 shows a gait cycle. The gait cycle (or stride) comprises two main phases: the stance P1 and the swing P2. Each of these phases comprises several subphases: P1 comprises the initial contact P11, the loading response P12, the mid stance P13, the terminal stance P14 and the pre-swing P15 while P2 comprises the initial swing P21, the mid swing P22 and the terminal swing P23.

**Figure** 2 shows an insole comprising a controller and a plurality of sensing cells.

**Figures 3A-3C** are graphs representing the latency of sensing cells. Fig. 3A shows the normalized pressure applied to a capacitive pressure sensing cell. Fig. 3B shows the normalized signal measured by the sensing cell. Fig. 3C is a zoom of Fig. 3B.

Figure 4 represents the method of determining toe off according to one embodiment of the present invention.

Figure 5 represents the method of identifying a discharge period according to one embodiment of the present invention.

Figure 6 represents the method of determining heel strike according to one embodiment of the present invention.

**DETAILED DESCRIPTION**

A method 100 according to the present invention allows to determine the toe off. The toe off is a moment separating two consecutive phases of the gait cycle (or stride) represented in figure 1: P15 and P21. The toe off is thus the exact moment when the foot is no longer in contact with the ground leading to a null pressure on the sole of said foot.

Advantageously, the method 100 determines the toe off with an insole 1 comprising a plurality of sensing cells 10 (an example of insole which may be used with the method 100 is shown in figure 2). This is advantageous because the insole 1 is located between the sole of the foot and the ground. Locating sensing cells 10 within the insole 1 thus allows, when the insole 1 is worn and used by a user during a gait, to measure the load (pressure or force) applied to the sole of the user and thus to determine when the foot is no longer in contact with the ground by using the method 100.

For example, the insole 1 may be the insole disclosed in WO2017033036. Advantageously, the sensing cells 10 are distributed along the surface of the insole 1. This is advantageous because the distribution of the load along the surface of the insole 1 can be computed with a higher precision leading to a higher precision of the method 100. Moreover, the higher the number of sensing cells, the lower the error on the values computed from the overall sensing cells 10. Preferably, the insole 1 comprises a number of sensing cells 10 comprised in a range between 5 and 30, more preferably between 15 and 20.

In one embodiment, at least one of the sensing cells 10 is located in the toe part 2 of the insole 1 (figure 2). This is advantageous in normal gait, where the toes are the last contact with the ground during P15 (figure 1). Indeed, since the toe off is the moment when the foot does not contact the ground anymore, locating at least one sensing cell in the toe part 2 of the insole 1 allows to compare the predetermined toe off threshold to a total signal to which at least the toe part 2 of the insole 1 contributes.

In one embodiment, at least one of the sensing cells 10 is located in the heel part 3 of the insole 1 (figure 2). This is advantageous in abnormal gait, where the toes are not the last contact with the ground during P15 (figure 1). Indeed, in abnormal gait, locating at least one sensing cell in the heel part 3 of the insole 1 allows to compare the predetermined toe off threshold to a total signal to which at least the part of the insole 1 other than the toe part 2 contributes.

The sensing cells 10 may be, for example, a pressure sensing cell or a force sensing cell. If the sensing cell is a pressure sensing cell, the sensing cell may be, for example, a capacitive pressure cell, a resistive pressure cell or a piezoelectric pressure cell. Capacitive pressure cells may comprise a thin sheet of deformable dielectric material placed between two electrodes. Indeed, the value of the capacitance C of a capacitive pressure cell can be determined as a function of the thickness L of the dielectric sheet of the capacitive pressure cells, the surface S of the upper electrode and the lower electrode of the capacitive pressure cells and the dielectric constant $\varepsilon$ of the material between the electrodes by the following equation: $C=\varepsilon S/L$. Consequently, when the thickness L of the dielectric sheet of the capacitive pressure cells is changed, the capacitance C varies. Change of thickness may be induced by the pressure applied by the user on the insole 1 while walking or exercising. If the sensing cell is a force sensing cell, the sensing cell may be, for example, a resistive force sensing cell - also known as force-sensing resistors FSR. A resistive force sensing cell is a sensor whose resistance changes when a force, pressure or mechanical stress is applied. It may be composed of superimposed layers arranged as: two substrate layers surrounding a conductive film and a plastic spacer, which includes an opening aligned with the conductive film. Above the plastic spacer there may be a conductive substrate. When an external force is applied to the sensing cell, the conductive film is deformed towards the substrate. Air from the opening of the plastic spacer is pushed through an air vent, and the conductive film comes into contact with the conductive substrate. The more of the conductive substrate gets in touch with the conductive film, the lower the resistance. Therefore, the more pressure applied on the sensor, the more the conductive substrate touches the conductive film making the resistance go down.

The insole 1 used for the method 100 also comprises a controller 15 to collect the measurements performed by the sensing cells 10 and/or to perform the computations associated to the method 100. The controller 15 may include a processor. The expression "processor" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

Preferably, the insole 1 is worn and used (by example, during a gait) by the user during the overall application of the method 100. In an alternative embodiment, the measurements from the sensing cells 10 are recorded by the controller 15 while the insole 1 is worn and used by the user and the method 100 is performed after the measurement session.

The signal measured by each sensing cell 10 may be pre-processed. The pre-processing may include a filtering, typically a smoothing filtering, so that high frequency variations in signals during stance are removed.

Preferably, the signal measured by each sensing cell 10 is a series of timestamped data, i.e., each signal is a succession of discrete data characterized by temporal information (measurement time) identifying the time when the data is measured. In another embodiment, the measurement session is divided in several measurement periods, each measurement period defining one measurement time. The overall data measured during a measurement period are then timestamped by the measurement time associated to said measurement period.

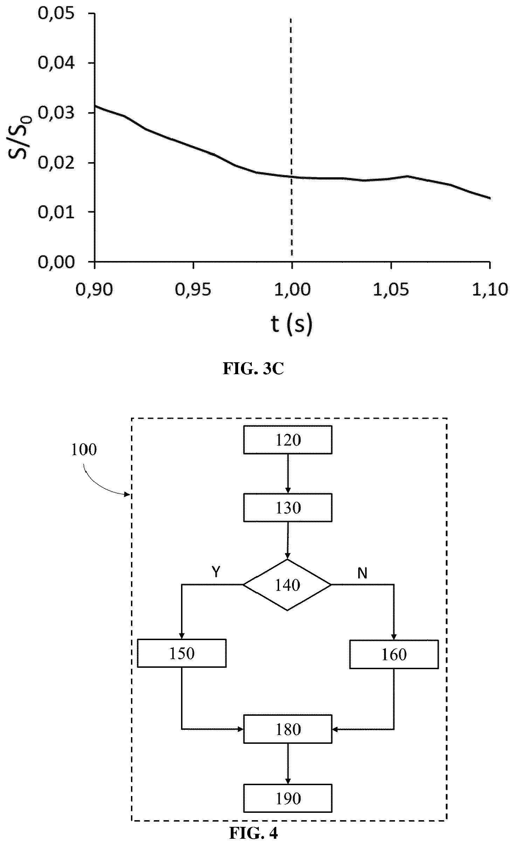

A first step 120 of the method 100 (figure 4) is the identification of a discharge period. The discharge period is a time period wherein the load applied to the sensing cells 10 is globally decreasing. This identification is advantageous because, during a stride, the load applied to a foot of the user is decreasing between P14 and P21 (figure 1). Since the toe off occurs between these two phases, identifying a discharge period allows to determine the time range during which the toe off will occur.

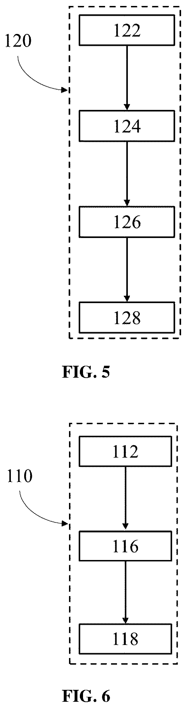

In one embodiment, the step 120 may comprise several steps represented in figure 5. First, considering that the sensing cells 10 have performed measurements during at least two measurement times, the controller collects the measured signal from each sensing cell 10 (step 122) for one measurement time, labelled t'. Preferably, the collected signals correspond to at least the second measurement time.

The total signal may be then computed from the signals collected from the overall sensing cells 10 for said measurement time t' (step 124). The total signal may be a simple sum of the signals output for the overall sensing cells 10. Alternatively, the total signal may be the result of the integration of a spatial interpolation of the signals collected from the overall sensing cells 10 as the method explained in the European patent application EP 20 306 521.4.

The total signal of the measurement time t' may be then compared to a total signal computed at a preceding instant t'-N$\tau$ (step 126), where $1/\tau$ represents the frequency of signal acquisition. Preferably, the total signal of the measurement time t' is compared to a total signal computed at the time just before, i.e., at the measurement time t'-$\tau$.

From this comparison, the determination of the beginning of the discharge period may be triggered when the total signal computed at the preceding instant t'-N$\tau$ is larger than the total signal of the current measurement time t' (step 128).

To avoid any false triggering due to the noise of each sensing cell which may imply a total signal computed at the preceding instant t'-N$\tau$ which is larger than the total signal of the measurement time t' while being out of the actual discharge period (this effect can occur mainly during P13 and P14 in figure 1), the signal measured by each sensing cell 10 and/or the total signal may be pre-processed. The pre-processing may include a filtering, typically a smoothing filtering, so that high frequency variations in signals during stance are not interpreted as a discharge.

Another way to avoid any false triggering is to trigger the determination of the beginning of the discharge period when the total signal corresponds to a force lower than 300N. Indeed, the minimal weight of an adult is about 40 kilograms corresponding to 400N. Setting the force threshold to 300N allows to be sure that the weight of the user has begun to move on the other feet and thus that the decay measured between two successive instants actually corresponds to the discharge period.

Yet another way to avoid any false triggering is to determine heel strike (step 110) before the step 120 of identification of the discharge period. In one embodiment, step 110 comprises several steps represented in figure 6. First, the controller 15 may collect the measured signal from each sensing cell 10 (step 112). The total signal may be then computed from the signals collected from the overall sensing cells 10 (step 116) associated to the same measurement time. The total signal may be a simple sum of the signals output for the overall sensing cells 10. Alternatively, the total signal may be the result of the integration of a spatial interpolation of the signals collected from the overall sensing cells 10 as the method explained in the European patent application EP 20 306 521.4. Finally, the heel strike may be triggered when the total signal exceeds a predetermined heel strike threshold (step 118). Advantageously, the step 110 allows to determine the initial contact during a stride. After the initial contact, the load on the sole of the foot contacting the ground increases until a maximum occurring during P13 and P14 (figure 1) before decreasing. Determining the heel strike thus avoids to trigger the determination of the beginning of the discharge period before the foot is contacting the ground because of the internal noise of each sensing cell 10 which may imply a total signal computed at the preceding instant t'-N$\tau$ which is larger than the total signal of the measurement time t'. In addition, the signal measured by each sensing cell 10 and/or the total signal may be pre-processed. The pre-processing may include a filtering, typically a smoothing filtering, so that high frequency variations in signals during stance are not misinterpreted.

In one embodiment, the predetermined heel strike threshold is a signal value corresponding to a force ranging between 5 and 40N, preferably between 10 and 30N, even more preferably between 15 and 25N. The conversion between a value of the force and a signal measured by a sensing cell 10 may be performed thanks to the transfer function, determined during the calibration phase, between at least one signal measured by the sensing cell 10 and corresponding force applied. Therefore, the force values of each sensing cell 10 may be computed in order to determine the total force which will be compared to the predetermined heel strike threshold.

Once the discharge period is identified, the controller 15 of the insole 1 may collect, during a step 130, the signal measured by each sensing cell 10. The collected signals may then be analyzed by the controller 15 during a step 140 by comparing the signal of each sensing cell 10 to a predetermined cell calibration threshold.

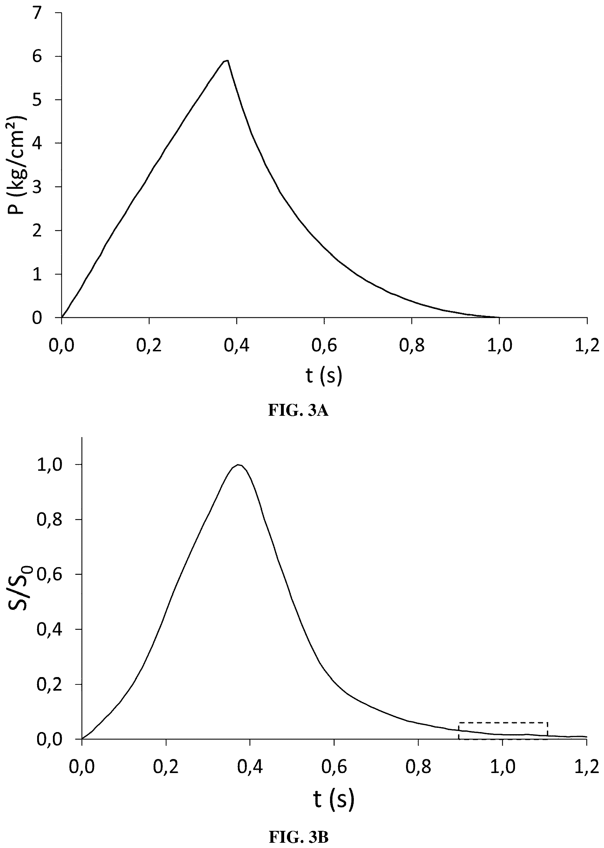

The predetermined cell calibration threshold is determined during the calibration phase for each sensing cell 10. In other words, each sensing cell 10 is associated with its own predetermined cell calibration threshold which may be different from those of the other sensing cells. During the calibration phase, a load may be applied to a sensing cell 10 and then decreased (unloading phase) until the load becomes null. Preferably, as shown in figure 3A, the unloading phase is configured to represent the decay of the load after P15. In alternative embodiments, the unloading phase is configured to represent any other behavior of a decay of the load. For pressure sensing cells, the load is a controlled pressure. For force sensing cells, the load is a controlled force.

In one embodiment, to determine the calibration curve, at least one data from the signal measured by said sensing cell 10 is associated to the value of the corresponding load - a pressure or a force. The predetermined cell calibration threshold is then determined as the signal measured by said sensing cell 10 when the load becomes null. In some embodiments, the predetermined cell calibration threshold may be determined by interpolation or as the signal corresponding to the first signal measured when the load becomes null.

In a specific embodiment, the calibration phase is performed by measuring a dynamic response of a sensing cell 10 with a load decreasing from its maximum value to zero in 0.12 to 0.7 s, corresponding to actual gait behavior, from low pace (long time for load decrease) to rapid pace (short time for load decrease). Said sensing cell 10 is then characterized by several calibration curves depending on the variation rate of the applied load. To determine each calibration curve, at least one data from the signal measured by said sensing cell 10 for each variation rate of the load is associated to the value of the corresponding load. The method of dynamic calibration may be, for example, the method disclosed in the European patent application EP 21 306 275.5. The predetermined cell calibration threshold may be then determined for each calibration curve as the signal measured by said sensing cell 10 when the load becomes null. In some embodiments, the predetermined cell calibration threshold associated to a variation rate of the load is determined, for said variation rate of the load, by interpolation of the corresponding calibration curve or as the signal corresponding to the first signal measured when the load becomes null.

In this specific embodiment in combination with pressure sensing cells, the applied load is a controlled pressure varying with a rate ranging from 5 to 30 $kg.cm^{-2}.s^{-1}$, preferably from 10 to 20 $kg.cm^{-2}.s^{-1}$.

In this specific embodiment in combination with force sensing cells, the applied load is a controlled force varying with a rate ranging from 600 to 10000 $N.s^{-1}$, preferably from 1400 to 4200 $N.s^{-1}$.

The predetermined cell calibration threshold may be a signal value corresponding to a pressure ranging between 0.05 and 0.5 $kg/cm^2$, preferably between 0.15 and 0.3 $kg/cm^2$. The conversion between a value of the pressure and a signal measured by a sensing cell 10 may be performed thanks to the transfer function, determined during the calibration phase, between the signal measured by the sensing cell and the pressure applied.

At a step 150, for each sensing cell 10 for which the signal is larger than or equal to the predetermined cell calibration threshold, the controller outputs the signal measured by said sensing cell(s) 10. Advantageously, for all other sensing cell(s) 10 (i.e., those for which the measured signal is lower than the predetermined cell calibration threshold), the controller outputs a null signal (step 160). This is advantageous because this allows to suppress most of the noisy signal produced, among other things, by the latency of the sensing cells allowing a method which is more precise.

This is illustrated in figure 3 with capacitive pressure cells comprising dielectric materials that are subject to latency occurring during the decay of the pressure applied to the capacitive pressure cell. Figure 3A shows the pressure P (expressed in kilograms per square centimeter) applied to a capacitive pressure cell. The pressure P is varied with time t (expressed in seconds) as it would be measured during a normal gait. After about 400 milliseconds, the pressure P applied to the capacitive pressure cell is decreased during 600 milliseconds in the same way than after P14 - the time scale of figure 3 is for illustration only: in normal gait, depending on gait pace, pressure decreases over a shorter time, typically from 250 to 300 milliseconds. Figure 3B shows the normalized signal measured by said capacitive pressure cell. In this example, the signal S is normalized by the maximal signal $S_0$ measured by said capacitive pressure cell. The signal $S/S_0$ measured at the end of the decreasing pressure period (pointed out by a dashed box) is zoomed in figure 3C. The vertical dashed line in figure 3C represents the time at which the pressure P is null (at 1 second). The latency of the capacitive pressure cell is clearly visible in this figure by the positive signal $S/S_0$ still measured by the capacitive pressure cell when the applied pressure P is null. If this measured signal S is used to determine the toe off, this will lead to an error up to several tens of milliseconds on its determination. The method 100 of the present invention is thus advantageous because outputting a null signal when the measured signal is lower than a predetermined cell calibration threshold allows to correct from the latency for each sensing cell 10 by suppressing the positive signal still measured by the sensing cell 10 when the applied pressure is null. This approach can also be applied for a force sensing cell, a resistive pressure cell or a piezoelectric pressure cell.

After the controller 15 has output the measured signals from the overall sensing cells, the total signal of the sensing cells is computed (step 180). The total signal may be a simple sum of the signals output for the overall sensing cells and for the same measurement time. Alternatively, the total signal may be the result of the integration of a spatial interpolation of the signals output for the overall sensing cells 10 at the same measurement time, for example using the method explained in the European patent application EP 20 306 521.4.

Finally, the toe off is triggered when the total signal is lower than a predetermined toe off threshold (step 190).

In one embodiment, the predetermined toe off threshold is a signal value corresponding to a force ranging between 5 and 40N, preferably between 10 and 30N, even more preferably between 15 and 25N. The conversion between signals measured by all sensing cells 10 and the value of the total force may be performed in various ways. In an embodiment, thanks to the transfer function between the signal measured by the sensing cell and the force applied, determined during the calibration phase, the force values of each sensing cell 10 are computed in order to determine the total force which will be compared to the predetermined toe off threshold. In another embodiment, the signals measured by all sensing cells 10 are pressure signals and may be spatially interpolated in a continuous pressure field, then integrated over the surface of the insole to yield the total force, as taught in European patent application EP 20 306 521.4.

In the embodiment where the method 100 is performed after the measurement session, the method 100 may be performed at once after the loading of the timestamped signal measured by the overall sensing cells 10. For example, the method 100 optionally starts with the step 110 for the determination of the heel strike by computing the total signal for each measurement time. The heel strike may be triggered as the first measurement time of a stride when the total signal exceeds the predetermined heel strike threshold. The discharge period is then identified (step 120) by comparing the total signal for each measurement time to the total signal at a preceding measurement time. The beginning of the discharge period may be triggered as the first measurement time of a stride (optionally occurring after the heel strike) when the total signal is lower than those at the preceding measurement time and optionally corresponding to a force lower than 300N. Considering only the signals measured during the discharge period, the step 140 is performed by comparing the signal of each sensing cell 10 to a predetermined cell calibration threshold. The step 150 or 160 is then performed according to the result of this comparison. The step 180 is then performed, by computing the total signal for each measurement time during the discharge period. The toe off is then triggered (step 190) as the first measurement time when the total signal is lower than a predetermined toe off threshold.

In the embodiment where the insole 1 is worn and used by the user during the overall application of the method 100, the method 100 may be performed at each measurement time after the signal measurement by the overall sensing cells 10. For example, considering the measurement time t, the method 100 optionally starts with the step 110 for the determination of the heel strike by computing the total signal measured at t. The heel strike t1 is then triggered when the total signal exceeds the predetermined heel strike threshold. The beginning of the discharge period t2 is then triggered when the total signal measured at a time t (optionally greater than t1) is lower than those at the preceding measurement time (t-N$\tau$) and optionally corresponding to a force lower than 300N. At t>t2, the step 140 is performed by comparing the signal of each sensing cell 10 to the corresponding predetermined cell calibration threshold. The step 150 or 160 is then performed according the result of this comparison. Still on said time t>t2, the step 180 is performed by computing the total signal and the toe off is triggered (step 190) when the total signal is lower than a predetermined toe off threshold.

The invention may also relate to a computer program product comprising instructions which, when executed by a computer, cause the computer to carry out the method 100 as disclosed above.

The expression "computer" should not be construed to be restricted to hardware capable of executing software, and refers in a general way to a processing device, which can for example include a computer, a microprocessor, an integrated circuit, or a programmable logic device (PLD). The computer may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

## EXAMPLE

The present invention is further illustrated by the following comparison performed between the stance duration measured using the method of the present invention (INV) and using a state of the art (SOA) method. Both methods were applied using an insole comprising 18 sensing cells, as shown in Figure 2.

The method of the present invention comprises the steps of determining heel strike (associated to the beginning of the stance) with a predetermined heel strike threshold of 20N, identifying a discharge period, and, during the discharge period, triggering output of the toe off (associated to the end of the stance) with a predetermined toe off threshold of 20N.

In the SOA method, the beginning of the stance is defined when the total load applied to the sensing cells of the insole becomes greater than a threshold of 20N and the end of the stance when the total load applied to the sensing cells of the insole becomes null. The duration of the stance is thus the difference between the end and the beginning of the stance.

In both methods (INV and SOA), the user is wearing shoes comprising the insole and walks on a BP400600 Biomechanics Force Platform supplied by AMTI. The force sensed by the platform is considered as the reference to determine the actual beginning and end of the stance.

Five users - without gait impairment - were asked to walk at low, normal and high speed. For each user and each walking pace, 60 stances were recorded, yielding a total of 900 stances representative of different gaits, and analyzed for each method. The experiment was reproduced with four insoles comprising different dielectric materials in their capacitive pressure cells. Table 1 below reports results obtained. For each insole, statistics over the 900 stances are reported for the method of the present invention and of the state of the art. The mean relative absolute error on the

$$\frac{\sum_{i=1}^{n} |fp_i - in_i| / fp_i}{n}$$

duration of the stance is defined as , where $fp_i$ is the measure obtained by the force platform and $in_i$ is the measure obtained with the insole. The bias of stance duration is the average difference between force platform

$$\frac{\sum_{i=1}^{n} |fp_i - in_i|}{n}$$

and insole stance duration and is defined as . Last, the upper limit of the 95% confidence interval - i.e., bias plus 1.96 standard deviation - is reported for the difference between force platform and insole stance duration measures.

Table 1

| Dielectric Material | INV/SOA | Mean relative absolute error (%) | Bias (ms) | Upper limit of the 95% confidence interval (ms) |
|---|---|---|---|---|
| 1 | SOA | 15.5 | 226 | 463 |
| 1 | INV | 5.2 | 80 | 184 |
| 2 | SOA | 12.5 | 195 | 429 |
| 2 | INV | 6.4 | 101 | 266 |
| 3 | SOA | 5.7 | 82 | 205 |
| 3 | INV | 4 | 59 | 135 |
| 4 | SOA | 10.9 | 145 | 375 |
| 4 | INV | 2.2 | 35 | 84 |

The four materials used for the dielectric material of the sensing cells are: Material 1: Polyurethane A - Material 2: Silicone A - Material 3: Silicone B - Material 4: Polyurethane B.

For the four types of dielectric materials, results show that the method according to the invention allows a much more precise determination of stance duration. The mean absolute relative error is significantly decreased, as well as the bias and the upper limit of the 95% confidence interval. In addition, measurements with insoles do not require the specialized laboratory equipment of a force platform, but can be performed in every day conditions, when a user is wearing insoles.

**Claims**

1. A method (100) for determining toe off with an insole (1) comprising a controller (15) and a plurality of sensing cells (10), the method comprising:

   - Identifying (120) a discharge period,
   - During the discharge period:

      o Collecting (130) by the controller (15) a measured signal from each sensing cell (10),
      o Outputting by the controller (15), for each sensing cell (10):

         ■ The signal if said signal is larger than or equal to a predetermined cell calibration threshold,
         ■ A null output if said signal is lower than said predetermined cell calibration threshold,

      o Computing (180), from the overall output signals, a total signal of the sensing cells (10), and
      o Triggering (190) output of the toe off when the total signal is lower than a predetermined toe off threshold,

   wherein the predetermined cell calibration thresholds are values determined during the calibration phase for each sensing cell (10) as the signal measured by said sensing cell (10) when an applied load becomes null during an unloading phase.

2. The method according to claim 1, wherein the predetermined toe off threshold corresponds to a force ranging between 5 and 40N.

3. The method according to claim 1 or 2, wherein identifying (120) a discharge period comprises:

   - Collecting (122) by the controller (15) a measured signal from each sensing cell (10),
   - Computing (124) a total signal from the measured signals,
   - Comparing (126) the total signal and a total signal computed at a preceding instant, and
   - Triggering (128) the determination, from this comparison, of the beginning of the discharge period when the total signal computed at the preceding instant is larger than the total signal.

4. The method according to claim 3, wherein, triggering (128) the determination of the beginning of the discharge period is performed when the total signal corresponds to a force lower than 300N.

5. The method according to any one of claims 1 to 4, further comprising, before identifying (120) a discharge period, determining (110) heel strike comprising:

   - Collecting (112) by the controller (15) a measured signal from each sensing cell (10),
   - Computing (116) a total signal from the collected signals, and
   - Triggering (118) the determination of a heel strike when the total signal exceeds a predetermined heel strike threshold.

6. The method according to claim 5, wherein the predetermined heel strike threshold corresponds to a force ranging between 5 and 40N.

7. The method according to any one of claims 1 to 6, wherein the total signal is computed as the sum of the signals outputted by the controller (15).

8. The method according to any one of claims 1 to 6, wherein sensing cells (10) measure a pressure and wherein the total signal is the integration of a field of pressure, said field of pressure being a spatial interpolation of pressure signals outputted by the controller (15).

9. The method according to any one of claims 1 to 6, wherein at least one sensing cell (10) is a capacitive pressure cell or a force sensing cell.

10. The method according to any one of claims 1 to 9, wherein calibration phase is performed by measuring a dynamic response of sensing cell (10) with a load decreasing from its maximum value to zero in 0.12 to 0.7 seconds.

11. The method according to any one of claims 1 to 10, wherein the sensing cells (10) are distributed along the surface of the insole (1).

12. The method according to any one of claims 1 to 11, wherein at least one of the sensing cells (10) is located in the toe part (2) of the insole (1).

13. The method according to any one of claims 1 to 12, wherein at least one of the sensing cells (10) is located in the heel part (3) of the insole (1).

14. A computer program product comprising instructions which, when the program is executed by a controller, cause the controller to carry out the steps of the method according to any one of claims 1 to 13.

P11    P12    P13    P14    P15    P21    P22    P23

P1

P2

**FIG. 1**

1    2

10

15

3

**FIG. 2**

**FIG. 3A**

**FIG. 3B**

**FIG. 3C**

**FIG. 4**

120

| 122 |
| --- |

↓

| 124 |
| --- |

↓

| 126 |
| --- |

↓

| 128 |
| --- |

**FIG. 5**

110

| 112 |
| --- |

↓

| 116 |
| --- |

↓

| 118 |
| --- |

**FIG. 6**

**EP 4 173 514 A1**

# EUROPEAN SEARCH REPORT

Application Number

EP 21 30 6522

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIDSTONE DANIEL E ET AL: "Electronic measurement of plantar contact area during walking using an adaptive thresholding method for Medilogic pressure-measuring insoles", THE FOOT, ELSEVIER, AMSTERDAM, NL, vol. 39, 17 January 2019 (2019-01-17), pages 1-10, XP085876938, ISSN: 0958-2592, DOI: 10.1016/J.FOOT.2019.01.009 [retrieved on 2019-01-17] | 1,2,5-14 | INV. A43B3/34 A43B3/44 A61B5/11 A61B5/00 G06V40/20 A43B17/00 |
| A | * pages 1-10 * | 3,4 | |
| A | US 2020/214595 A1 (ROBERTS JASON [GB] ET AL) 9 July 2020 (2020-07-09) * abstract * * paragraphs [0105], [0106], [0110], [0125], [0126] * | 1-14 | |
| A | WO 2021/213634 A1 (ECOLE POLYTECHNIQUE FED DE LAUSANNE EPFL TTO [CH]) 28 October 2021 (2021-10-28) * abstract * * page 3, line 5 - page 50, line 27 * * figures 1-13C * | 1-14 | TECHNICAL FIELDS SEARCHED (IPC) A61B G06V A43B G06K |

The present search report has been drawn up for all claims

| Place of search | Date of completion of the search | Examiner |
|---|---|---|
| The Hague | 12 April 2022 | Espeel, Els |

EPO FORM 1503 03.82 (P04C01)

**ANNEX TO THE EUROPEAN SEARCH REPORT
ON EUROPEAN PATENT APPLICATION NO.**

EP 21 30 6522

This annex lists the patent family members relating to the patent documents cited in the above-mentioned European search report.
The members are as contained in the European Patent Office EDP file on
The European Patent Office is in no way liable for these particulars which are merely given for the purpose of information.

12-04-2022

| Patent document cited in search report | Publication date | Patent family member(s) | Publication date |
|---|---|---|---|
| US 2020214595 A1 | 09-07-2020 | NONE | |
| WO 2021213634 A1 | 28-10-2021 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017033036 A
- EP 20306521 A
- EP 21306275 A